# EUROPEAN PATENT SPECIFICATION

(11) **EP 4 403 190 B1**
(45) Date of publication and mention of the grant of the patent: **06.05.2026**
(21) Application number: 22857949.6
(22) Date of filing: 05.08.2022
(51) Int. Cl.: A61K 9/50, A61K 9/51, A61K 48/00, B82Y 5/00, A61K 47/62, A61K 47/69, C12N 15/87

(54) **SILICA PARTICLES FOR ENCAPSULATING NUCLEIC ACIDS**
KIESELSÄURETEILCHEN ZUR VERKAPSELUNG VON NUKLEINSÄUREN
PARTICULES DE SILICE POUR L'ENCAPSULATION D'ACIDES NUCLÉIQUES

(30) Priority: 20.08.2021 ES 202130804
(43) Date of publication of application: 24.07.2024
(73) Proprietor: Fundación Instituto de Investigación Marqués de Valdecilla (IDIVAL), 39011 Santander (Cantabria) (ES)
(72) Inventor: LÓPEZ FANARRAGA, Mónica, 39011 Santander (Cantabria) (ES); MARÍN CABA, Laura, 39011 Santander (Cantabria) (ES)
(74) Representative: Pons IP
(86) International application number: PCT/ES2022/070528
(87) International publication number: WO 2023/021230

(56) References cited:
- US-A1- 2016 067 191
- KAPUSUZ DERYA ET AL: "Synthesis of DNA-encapsulated silica elaborated by sol-gel routes", JOURNAL OF MATERIALS RESEARCH, vol. 28, no. 2, 28 September 2012 (2012-09-28), US, pages 175 - 184, XP093292439, ISSN: 0884-2914, DOI: 10.1557/jmr.2012.309
- KAPUSUZ DERYA: "Sol-gel derived silica/polyethylene glycol hybrids as potential oligonucleotide vectors", JOURNAL OF MATERIALS RESEARCH, vol. 34, no. 22, 28 November 2019 (2019-11-28), US, pages 3787 - 3797, XP055894358, ISSN: 0884-2914, DOI: 10.1557/jmr.2019.341
- ZHANG YURAN, MANLEY TIMOTHY SPENCER, LI KEWEN, HORNE ROLAND N: "DNA-Encapsulated Silica Nanoparticle Tracers for Fracture Characterization", GRC TRANSACTIONS, vol. 39, 1 January 2015 (2015-01-01), pages 967 - 974, XP093038669
- PAUNESCU D ET AL.: "Reversible DNA encapsulation in silica to produce ROS-resistant and heat-resistant synthetic DNA ' fossils&apos", NATURE PROTOCOLS, vol. 8, no. 12, 30 November 2012 (2012-11-30), pages 2440 - 2448, XP055203976, ISSN: 1754-2189, DOI: 10.1038/nprot.2013.154
- PARIS JUAN L., VALLET-REGÍ MARÍA: "Mesoporous Silica Nanoparticles for Co-Delivery of Drugs and Nucleic Acids in Oncology: A Review", PHARMACEUTICS, vol. 12, no. 6, pages 526, XP093038670, DOI: 10.3390/pharmaceutics12060526
- KANJIRO MIYATA, NOHA GOUDA, HIROYASU TAKEMOTO, MAKOTO OBA, YAN LEE, HIROYUKI KOYAMA, YUICHI YAMASAKI, KEIJI ITAKA, NOBUHIRO NISHIY: "Enhanced transfection with silica-coated polyplexes loading plasmid DNA", BIOMATERIALS, ELSEVIER, AMSTERDAM, NL, vol. 31, no. 17, 20 March 2010 (2010-03-20), AMSTERDAM, NL , pages 4764 - 4770, XP055328954, ISSN: 0142-9612, DOI: 10.1016/j.biomaterials.2010.02.033
- STOBER W., FINK A., BOHN E.: "CONTROLLED GROWTH OF MONODISPERSE SILICA SPHERES IN THE MICRON SIZERANGE.", JOURNAL OF COLLOID AND INTERFACE SCIENCE, ACADEMIC PRESS,INC., US, vol. 26., 1 January 1968 (1968-01-01), US , pages 62 - 69., XP000561462, ISSN: 0021-9797, DOI: 10.1016/0021-9797(68)90272-5

## Description

The present invention belongs to the technical field of nanomedicine and nanotechnology, specifically to particles for gene transfer. The present invention relates to silica particles comprising nucleic acids encapsulated inside the same. Furthermore, the present invention relates to a method for producing said particles and the uses thereof in gene transfer or cell marking and as a medicinal product, specifically as a medicinal product for protein/enzyme replacement therapy and as an immunisation system.

### BACKGROUND OF THE INVENTION

At present, there is a growing interest in the development of systems that allow the vectorisation or distribution of nucleic acids. Among the applications of these systems, various therapeutic approaches stand out, such as the use thereof in the treatment of genetic diseases, in which gene transfer makes it possible to correct or replace genes that cause certain diseases in the tissues and cells of patients.

Another therapeutic approach based on the distribution of nucleic acids encompasses the production of vaccines, for example, the SARS-CoV-2 vaccine against COVID19, which is based on a system for the gene transfer of lipid particles that encapsulate mRNA encoding the 'S' protein of the virus backbone, which is critical for its binding to human cells. However, the distribution of biologically active agents, such as nucleic acids, to cells and tissues, presents associated technical difficulties, among others, its stability having a limited duration.

At present, methods used for nucleic acid distribution include viral and non-viral vectors. Viral vectors have high efficiency in the distribution of genetic material to cells, being systems widely used in biomedicine. Among them, the use of vectors derived from adeno-associated viruses especially stands out, which is widely used in gene therapy due to its biosafety, low toxicity and selective tropism. Nevertheless, viral vectors have problems for their clinical application, such as limitations in the size of the gene to be transduced, lack of specificity, immunogenicity and possible oncogenic effects.

In turn, the use of non-viral vectors overcomes many of these limitations, with there being a growing interest in systems for gene transfer of this type. The most commonly used systems for the distribution of nucleic acids comprise positively charged carrier molecules that neutralise the negative charge of nucleic acids. Among them, cationic liposomes, which do not present limitations in the size of the DNA to be transduced, have low immunogenicity and toxicity, and can be managed *in vivo,* intravenously, with the particles generally being retained mechanically in natural filters, such as the lungs and liver, or intramuscularly, stand out. Nevertheless, their low stability and rapid degradation in the body pose barriers to their logistical distribution and clinical implementation.

New strategies have been developed in fields such as nanomedicine for the design and production of new systems capable of vectorising nucleic acids with greater potential. Nanomedicine generally refers to the medical application of nanotechnology, an interdisciplinary field that exploits the distinctive characteristics of materials, the molecular and cellular size range of which, as well as the versatility thereof, make them systems of interest for the distribution of drugs and nucleic acids.

Specifically, the particles show outstanding properties as systems for gene transfer, among others, the ability to target specific tissues or cells, protection against nuclease degradation, improvement of DNA stability and increased transformation efficiency and safety. In fact, they are increasingly more accepted from a clinical point of view as they are considered one of the most promising vectorisation systems due to their biological behaviour and versatility in shapes and sizes (Chen et al., 2016, Molecular Therapy - Methods & Clinical Development, 3, 16023*).*

There are numerous examples of particles capable of vectorising or distributing different therapeutic compounds and nucleic acids for use in gene transfer (Dizaj *et al.,* 2014). Different types of particles have been developed for the distribution of nucleic acids: polymeric types (such as micelles, nanogels, linear polymers, dendrimers, polymersomes), inorganic types (such as gold particles, quantum dots, silica particles, carbon-based nanomaterials), liposomes, exosomes and nanostructure-based DNA carriers, as well as hybrids that integrate the advantages of different materials. All of these systems are primary structures that can in turn be combined with organic or inorganic ligands on the surface thereof to improve targeting or prevent capture by the macrophages of the mononuclear phagocytic system.

Nevertheless, most of these systems are very unstable and, to that end, must be prepared minutes before use or stored in extreme cold conditions, which complicates the logistics thereof at an industrial level and represents a loss of reliability and reproducibility.

Various studies are focusing on inorganic particles, and more specifically on silica particles (Li Tang and Jianjun Chen, Nanotoday, Vol 8, 3, 290-312 (2013)) due to their properties: they overcome some of the limitations of organic nanosystems, such as low stability in physiological conditions, and offer many advantages compared to other types of inorganic materials, such as a controllable size, ease of surface modification, ability to be produced on a large scale and being highly biocompatible. Among the methods used for the production of particles of this type, the *Stöber* method (Stöber, W., et al., J. Colloid Interface Sci. 26 (1), 62-69 (1968)), which is based on the hydrolysis of a silica precursor in an alcoholic solution in the presence of ammonia as a catalyst, and which, despite being proposed 40 years ago, is still widely used.

Surface modifications (on the outer or inner surface, in pores) of silica particles already formed by means of coating with positive charges, and subsequently electrostatically adding nucleic acids to said surfaces have been included in recent years, because pure silica particles have a negative charge and, therefore, difficulties in electrostatic interaction with nucleic acid molecules that are also negatively charged.

Kapusuz D., 2019, Journal of Materials Research 34(22): 3787-3797, discloses hybrid silica/ PEG particles encapsulating oligonucleotides.

There is therefore a need in the state of the art to develop alternative nucleic acid distribution systems that are stable, reproducible and efficient in gene transfer.

### DESCRIPTION OF THE INVENTION

The inventors of the present invention have developed silica particles (SiO₂) that are stable, reproducible and efficient in gene transfer because they comprise nucleic acids (NAs) encapsulated inside the same.

The methods described in the prior art modify the surface of the silica particles already formed by means of coating with positive charges, and subsequently electrostatically add nucleic acids to said surfaces. In contrast, the inventors have introduced the NAs into the precursor mixture of the silica particles, the nucleic acids thereby remaining integrated within the silica particle and only being released when the particle is dissolved, allowing the use thereof in gene transfer, cell marking or therapeutic use, for example, in protein/enzyme replacement therapy, as RNA transporters (for example RNA inhibitors or interference) or in the production of vaccines. This means that the NAs are highly protected and that, furthermore, the surface of the particles can be used for other purposes.

Silica is a completely biocompatible compound that does not produce any type of toxicity at usage doses, even when nanoparticles of this compound are captured and dissolved inside neurons (Iturrioz-Rodríguez N., et al. 2020, Pharmaceutics, 28;12(6):487)*.* Particles containing encapsulated nucleic acids can be stored for months in ethanol, because silica and nucleic acids are very stable in ethanol. Furthermore, particle synthesis can be easily scaled, allowing preparation in large quantities. This, added to the high stability thereof, allows the production and distribution of particles at an industrial level without losing effectiveness, even at room temperature for at least 1 month.

Therefore, in a first aspect, the present invention relates to a method of producing a silica particle comprising at least one nucleic acid encapsulated inside said particle, hereinafter "particle of the invention", the method being as defined in claim 1. In another aspect, the invention relates to a silica particle obtained by the method of claim 1, as defined in independent claim 9. In yet another aspect, the invention relates to the *in vitro* uses of the particles defined in independent claims 10 and 11, to the particles for use as a medicinal product as defined in independent claims 12 and 14, and to the use of the particle defined in independent claim 15. Further aspects of the invention are described in the dependent claims.

The term "silica particle" refers in the present invention to inorganic particles made up of silicon oxide or silica (SiO₂) and comprising dimensions in the range between 1 and 5000 nm, preferably with a spherical morphology. The particles of the invention form a colloidal system, colloidal suspension or colloidal dispersion comprising a fluid (liquid or gas) phase and another disperse phase in the form of solid silica particles. Preferably the suspension or colloidal system is a uniform or monodisperse suspension.

The particles of the invention are characterised by having a mean particle size equal to or less than 5 µm, preferably they have a mean diameter size between 1 and 2000 nm. This size allows the nanoparticles to penetrate cells, dissolve and administer the biologically active molecule, i.e., nucleic acids or NAs, terms used interchangeably throughout the present document.

In a preferred embodiment, the particle of the invention has a size between 1 nm to 1000 nm, more preferably between 200 nm and 500 nm in diameter.

In another even more preferred embodiment, the particle of the invention has a size that is selected from: 200 nm, 210 nm, 220 nm, 230 nm, 240 nm, 250 nm, 260 nm, 270 nm, 280 nm, 290 nm, 300 nm, 310 nm, 320 nm, 330 nm, 340 nm, 350 nm, 360 nm, 370 nm, 380 nm, 390 nm, 400 nm, 410 nm, 420 nm, 430 nm, 440 nm, 450 nm, 460 nm, 470 nm, 480 nm, 490 nm or 500 nm in diameter.

The silica particles (SiO₂) of the invention comprise nucleic acids (NAs) encapsulated inside the same, i.e., they are integrated within the silica particle and are only released when the particle is dissolved.

The term "encapsulation" as it is used in the present invention, refers to the provision, localisation or integration inside the particle of biological agents, specifically nucleic acids, so that they are physically inside the structure of the particle, thus leaving the nucleic acid molecule(s) encapsulated inside the silica particle.

The term "nucleic acid" or "nucleotide sequence", used interchangeably in the present invention, refers to a molecule or sequence of nucleic acids, specifically of deoxyribonucleotides (DNA), ribonucleotides (RNA) and/or modified versions thereof, as well as their polymers in single-stranded and/or double-stranded form, or their complements.

Therefore, in another preferred embodiment, the nucleic acid encapsulated in the particle is DNA and/or RNA.

Examples of nucleic acids include, but are not limited to, double-stranded linear DNA (dsDNA), messenger RNA (mRNA), plasmid DNA (pDNA), RNA interference (RNAi), hairpin RNA (shRNA), microRNA (miRNA), guide RNA (gRNA) or supercoiled DNA.

In another more preferred embodiment, the nucleic acid encapsulated in the particle of the invention is selected from the list consisting of: dsDNA, mRNA, pDNA, RNAi, shRNA, miRNA, gRNA and supercoiled DNA.

The silica particles of the invention are efficient in gene transfer because, once they penetrate inside the cell, they dissolve, releasing the elements encapsulated in said particles into the cell cytoplasm, specifically nucleic acids (NAs).

As understood by a person skilled in the art, the nucleic acid encapsulated in the particle of the invention, or the protein that encodes said NAs, can regulate cellular gene expression, specifically overexpressing, inhibiting or silencing cellular gene expression.

The terms "gene silencing" or "gene inhibition" used in the present invention, refer interchangeably to a cellular mechanism by means of which, by means of NAs or proteins encoded by the encapsulated NAs, gene expression is inhibited, controlling the expression of endogenous and exogenous genes, carried out at a post-transcriptional (PTGS) or transcriptional (TGS) level. Examples of proteins or NAs that are responsible for silencing expression include, but are not limited to, Dicer proteins, argonauts, nuclease (RNase or DNase), miRNA or siRNA.

Moreover, in addition to regulating gene expression, encapsulated NAs can encode cell locating or marking proteins, such as fluorescent proteins. This allows the particles of the invention to be used in the identification of cell structures by means of encapsulating NAs that encode cell markers.

Therefore, in another preferred embodiment, the nucleic acid encapsulated inside the particle of the invention encodes at least one cell marker.

The term "cell marker", as used herein, refers to a protein, such as a fluorescent or luminescent protein, or an enzyme, or a fragment thereof, or to a chemical molecule, the expression and/or activity of which can give rise to a detectable signal that can be visualised and which can therefore be used in cell marking to identify a cell or a cellular component or fraction (for example, cell surface, nucleus, organelle, cell fragment or other material that originates from or is part of a cell), cell lineages or lines, tissues or organs, as well as to characterize and identify a particular state of cells (for example a disease or physiological state such as apoptotic or non-apoptotic, a differentiated state or an undifferentiated state). Examples of cell markers include, but are not limited to, chromophores, fluorochromes, fluorophores such as fluorescein (FITC), *texas red,* phycoerythrin (PE), rhodamine, acridine orange, hoescht, DAPI, propidium iodide, ethidium bromide, Dio, Dil, DiR, allophycocyanin, hybridisation probes, antibody fragments, affinity tags (for example, biotin or avidin), enzymes such as alkaline phosphatase (AP) or horseradish peroxidase (HRP).

It is routine practice for a person skilled in the art to determine the presence, absence or level of expression of a cell marker using standard techniques known in the prior art.

Examples of cell marker visualisation or identification techniques include, but are not limited to, *Northern blot, in situ* hybridisation, RT-PCR, sequencing, immunological methods (immunoblotting, immunohistochemistry, fluorescence detection after staining with fluorescently labelled antibodies), cytometry, fluorescence microscopy, oligonucleotide microarrays or cDNA, protein microarray analysis or mass spectrometry.

In another preferred embodiment, the nucleic acid (NA) encapsulated in the particle of the invention encodes a fluorescent marker, preferably a fluorophore, more preferably a fluorescent protein.

Examples of fluorescent proteins include, but are not limited to, green fluorescent protein (GFP)(*GFP*, for its acronym in English), red fluorescent protein (RFP; or also mCherry), far-red fluorescent proteins (for example, TagRFP657), infrared-emitting fluorescent proteins (for example, miRFP709 or iRFP720), blue fluorescent protein (BFP), yellow fluorescent protein (YFP) or any combination thereof.

As understood by a person skilled in the art, encapsulated NAs may also comprise cellular localisation sequences, such as a nuclear localisation sequence (NLS) to import the protein into the nucleus, or subcellular localisation sequences for the importing thereof into mitochondria, peroxisomes, endoplasmic reticulum or chloroplasts, among others. This will localize the expression of the fluorescent protein to different subcellular locations.

Thus, in another preferred embodiment, the encapsulated nucleic acid encodes viral, bacterial or parasitic proteins or protein fragments.

The term "viral, bacterial, parasitic protein or protein fragment or peptide toxins of eukaryotic origin", as used herein, refers to a protein or fragment of a protein that makes up or is the product of a virus (for example, SARS-CoV-2, flu virus), bacteria (for example toxins such as Shiga or botulinum), parasite (for example *Anopheles* mosquitoes causing malaria) or of eukaryotic organisms (for example, peptide toxin of puffer fish, snail, insect or snake toxin).

Examples of viral proteins include, but are not limited to, structural proteins (capsid and viral envelope proteins), nonstructural proteins, regulatory proteins and accessory proteins.

In a more preferred embodiment, said protein or viral protein fragment is from SARS-CoV-2.

Examples of bacterial proteins include, but are not limited to, modified toxins or toxoids, adhesins, outer membrane proteins, flagellum proteins, periplasmic proteins and intracellular bacterial enzymes.

Examples of parasitic proteins include, but are not limited to, surface proteins, transmembrane proteins, aquaporins, cuticle proteins, glutathione S-transferases, serine proteases, aminopeptidases, secretion proteins, or factors that attenuate the host's immune response, such as the PMIF (plasmodium macrophage migration inhibitory factor) protein.

As understood by a person skilled in the art, encapsulated NAs may also comprise therapeutic gene sequences, among others, NAs that contain sequences encoding genes of cellular damage repair proteins or RNAs or "caretaker" genes, tumour suppressors or genes, or apoptosis-inducing genes, for example, in cancer treatment.

Thus, in another preferred embodiment, the encapsulated nucleic acid encodes at least one tumour suppressor gene or one cellular damage repair gene.

The term "tumour suppressor genes" as used in the present invention refers to a gene that reduces the probability that a cell in a multicellular organism will transform into a cancer cell and inhibit excessive cell proliferation, wherein proteins encoded by tumour suppressor genes stop cell cycle progression in response to DNA damage or growth suppression signals from the extracellular environment (contact inhibition). Examples of tumour suppressor genes include, but are not limited to, Retinoblastoma or RB1, TPP53 gene or Neurofibromatosis type 1 or 2 (NF1 or NF2), HNPCC (hereditary non-polyposis colon cancer) genes (MLH, MSH2 and MSH6), DCC (colorectal cancer) genes, CDKN2A, ARID1A, ATM, CHK2, APC, BRCA1 and BRCA2.

The term "cellular damage repair genes" or "caretaker genes" as used in the present invention refers to genes involved in the repair of DNA alterations and in maintaining the integrity of the genome and proteome. Examples of cellular damage repair genes include, but are not limited to, base excision repair (BER), nucleotide excision repair (NER) and mismatch repair (MMR) genes, genes that encode molecular chaperones (Hsp70, Hsp90, Hsp60), growth factors (NGF, EGRF, CNTF, BDNF), or RAF-MEK-ERK type signal cascade regulators.

As understood by a person skilled in the art, encapsulated NAs may also comprise gene sequences that activate or inhibit cellular repair in neurons, for example inhibiting the formation of toxic compounds or defective proteins such as amyloid.

As understood by a person skilled in the art, in addition to encapsulated NAs, the silica particle of the invention may comprise proteins forming gene editing systems, for example, the CRISPR-Cas system, although they are systems that comprise NA fragments, specifically guide RNA, accompanied by proteins, specifically nucleases.

The term "gene editing system" refers in the present invention to a type of genetic engineering in which manipulation, modification or direct alteration of a DNA sequence is carried out in the genome of a cell or organism either by eliminating, inserting or replacing a sequence of interest in the genotype thereof, specifically by means of using enzymes called nucleases, which specifically cut the DNA or genome. Examples of gene editing systems include, but are not limited to, ZFN (zinc finger nucleases), TALEN (transcription activator-like effector nuclease) and CRISPR (clustered regularly interspaced short palindromic repeats).

In another preferred embodiment, the particle of the invention comprises an encapsulated gene editing system, wherein the gene editing system comprises a nucleic acid and a nuclease.

In another more preferred embodiment, the particle of the invention comprises an encapsulated gene editing system, wherein the gene editing system is selected from the list consisting of: ZNF, TALEN or CRISPR.

In another even more preferred embodiment, the particle of the invention comprises an encapsulated gene editing system, wherein the gene editing system is CRISPR, more preferably wherein the CRISPR system is selected from the list consisting of: CRISPR-Cas9, CRISPR-Cas12a, CRISPR-CasΦ, CRISPR-Cas3, CRISPR-Cas8, CRISPR-Cas10 and CRISPR-Cas4, even more preferably CRISPR-Cas9.

As understood by a person skilled in the art, in addition to encapsulated NAs, the silica particle of the invention may comprise other molecules encapsulated inside the same (that are not encoded by NAs), such as magnetic nanoparticles, therapeutic molecules or drugs or cell markers (not encoded by NAs, such as radiological markers or radioisotopes) to promote their entry into the cell cytoplasm using magnetic force or the visualisation thereof using fluorescence techniques, respectively.

Thus, in another preferred embodiment, the particle of the invention further comprises encapsulated cell markers not encoded by NAs. The term "cell markers" has been described above and applies equally to this particular embodiment. Examples of cell markers (not encoded by NAs) include, but are not limited to, fluorescent probes with affinity for subcellular structures such as acidic vesicles (endosomes and/or lysosomes, for example, Lisotracker^{®}) or for nucleic acids (Hoescht or DAPI for DNA, ethidium bromide, propidium iodide, acridine orange, etc.), marker compounds of the cell cytoplasm or mitochondria or lipophilic compounds (Dil, DiO, DiR) to mark membranes.

Thus, in another preferred embodiment, the particle of the invention further comprises encapsulated magnetic nanoparticles. Examples of magnetic particles include, but are not limited to, particles formed by iron oxides, nickel and cobalt; or other elements that combine several metals, like zinc, copper, strontium or barium.

Thus, in another preferred embodiment, the particle of the invention further comprises therapeutic molecules or drugs. Examples of drugs include, alkylating agents (Altretamine; Bendamustine; Bulsufan; Carboplatin; Carmustine; Chlorambucil; Cisplatin; Cyclophosphamide; Dacarbazine; Ifosfamide; Lomustine; Mechlorethamine; Melphalan; Oxaliplatin; Temozolomide; Thiotepa; Trabectedin); Nitrosoureas (Carmustine; Lomustine; Streptozocin); anti-metabolites (zacitidine; 5-fluorouracil; 6-mercaptopurine; Capecitabine; Cladribine; Clofarabine; Cytarabine; Decitabine; Phloxiridine; Fludarabine; Gemcitabine; Hydroxyurea; Methotrexate; Nelarabine; Pemetrexed; Pentostatin; Pralatrexate; Thioguanine); anthracycline-type anti-tumour antibiotics (Daunorubicin; Doxorubicin; Epirubicin; Idarubicin; Valrubicin); or non-anthracycline antibiotics (Bleomycin; Dactinomycin; Mitomycin C; Mitoxantrone). Other compounds comprise topoisomerase inhibitors (Irinotecan; Topotecan; Etoposide; Mitoxantrone; Teniposide); mitosis inhibitors such as taxanes (Capazitaxel; Docetaxel; Paclitaxel); vinca alkaloids (Vinblastine; Vincristine; Vinorelbine); corticosteroids (Prednisone; Methylprednisolone; Dexamethasone) or other chemotherapy drugs such as Transretinoic acid; Arsenic trioxide; Asparaginase; Eribulin; Hydroxyurea; Ixabepilone; Mitotane; Omacetaxine; Pegasparaginase; Procarbazine; Romidepsin or Vorinostat.

The nucleic acids integrated within the silica particle of the invention are only released when the particle is dissolved, allowing the NAs to be highly protected and, furthermore, the surface of the particles can be functionalised for other purposes, such as, for example, for the targeting or coating of the particle of the invention, by means of protein coatings, antibodies and/or the binding of targeting ligands to the particle surface.

Thus, the particle of the invention further comprises at least one targeting ligand and/or a protein coating on its surface.

The term "targeting ligand" as used in the present invention refers to molecules or particles, of a biological origin (for example peptides, proteins, nucleotides) or of a physicochemical origin, which direct, focus or transport the particle of the invention to a certain cell type, tissue or organ.

Examples of targeting ligands include, but are not limited to, proteins or peptide ligands (e.g. neuron-binding peptides, viral or bacterial proteins and/or toxins), antibodies, nanoantibodies, dendrimers, molecules targeting receptors (for example, folic acid) or aptamers.

The term "protein coating" as used in the present invention refers to protein molecules (peptides, proteins or protein fragments) that when assembled and/or attached to the silica surface of the particles of the invention coat and form a cover that surrounds the silica particle of the invention in at least 75% of the total surface of the silica particle, more preferably 80 to 100% of the total surface.

Likewise, the targeting ligands and protein coating can be functionalised with cell markers, such as fluorescent molecules, to allow visualisation of said protein coating.

In another even more preferred embodiment, the protein coating or targeting ligand of the particle surface of the invention further comprises at least one cell marker.

The terms "cell marker", "targeting ligand" and "protein coating" have already been described in previous embodiments of the present invention and apply equally to this preferred embodiment.

Furthermore, the surface of the silica particle of the invention can in turn be coated with a coating made up of the same silica particles of the invention, obtaining particles with a double layer of SiO₂ and different encapsulated NAs. Thus, in another preferred embodiment, the particle of the invention comprises a silica coating on its surface where said silica coating comprises silica particles with encapsulated NAs, i.e., silica particles of the invention, forming a particle with a double layer of silica.

As understood by a person skilled in the art, the silica particle of the invention may be comprised in a composition for *in vitro* use, for example, in gene transfer or cell marking as described hereinbefore.

Therefore, another aspect of the invention is a composition comprising the silica particle of the invention, hereinafter "composition of the invention".

The silica particle has been described hereinbefore and applies equally to this aspect of the invention, as well as all its particular embodiments (alone or in combination).

Specifically, the composition of the invention may be a pharmaceutical composition for use as a medicinal product, for example, in protein/enzyme replacement therapy. Thus, in a preferred embodiment, the composition of the invention is a pharmaceutical composition.

The term "pharmaceutical composition" refers to a set, mixture, combination of components or substances that comprise the silica particle of the invention in any concentration. The pharmaceutical composition may be for human use. The term "pharmaceutical composition for human use" refers to a composition, substance or combination of substances with properties for use as a medicinal product, specifically as a medicinal product for protein/enzyme replacement therapy in humans or that can be used in humans or administered to humans in order to restore, correct, or modify physiological functions by exerting a pharmacological, immune or metabolic effect, or establish a medical diagnosis.

In another preferred embodiment, the pharmaceutical composition further comprises at least one pharmacologically acceptable carrier and/or excipient.

The term "vehicle" or "carrier", refers to a substance, preferably an inert substance, which facilitates the incorporation of other compounds, which allows a better dosage and administration or improves the consistency and form of the pharmaceutical composition for use as a medicinal product for protein/enzyme replacement therapy. Therefore, the carrier is a substance that is used in the medicinal product to dilute any of the components of the pharmaceutical composition to a certain volume or weight; or that even without diluting said components, it is capable of allowing a better dosage and administration or giving consistency and form to the medicinal product. When the form of presentation is liquid, the pharmaceutically acceptable carrier is the diluent.

The term "excipient" refers to a substance that aids the absorption of any of the components of the pharmaceutical composition, stabilizes said components, to modify the organoleptic properties thereof or to determine the physicochemical properties of the pharmaceutical composition and the bioavailability thereof. Thus, the excipients may have the function of holding the components together, such as, for example, starches, sugars or celluloses; the function of sweetening; the function of colouring; the function of protecting the medicinal product, such as, for example, to isolate it from the air and/or humidity; the function of filling a tablet, capsule, pill or any other form of presentation, such as, for example, dibasic calcium phosphate; the disintegrating function to facilitate the dissolution of the components; without excluding other types of excipients not mentioned herein.

Furthermore, as understood by a person skilled in the art, the excipient and the carrier must be pharmacologically acceptable. The term "pharmaceutically acceptable" refers to the vehicle or excipient having to allow the activity of the compounds of the pharmaceutical composition, in particular of the particle of the invention, i.e., it is compatible with said components, so that it does not cause harm to the organisms to which it is administered.

The pharmaceutical composition or medicinal product can be presented in any clinically permitted form of administration and in a therapeutically effective amount. For example, it may be in a form adapted for oral, sublingual, nasal, intrathecal, bronchial, lymphatic, rectal, transdermal, intravenous, intraperitoneal, orogastric, intracolonic, inhaled or parenteral administration thereof.

In another preferred embodiment, the pharmaceutical composition is a vaccine, preferably a vaccine against SARS-CoV-2.

The term "vaccine" as used herein refers to a particle or composition which, when administered to a subject, induces a cellular and/or humoral immune response. This may include an antigen or combinations of antigens. In the present invention, the antigen is encoded by the nucleic acid encapsulated in the particle of the invention.

The inventors of the present invention have developed silica particles, that are stable, reproducible and efficient in gene transfer because they have introduced nucleic acids (NAs) into the mixture of components that will form the silica particles.

To that end, another aspect of the invention relates to a method for producing the particle of the invention, hereinafter the "method of the invention", comprising the following steps:
(i) dissolving ethanol (EtOH) in a molar concentration of between 8 and 12 mol/L (M), ammonium hydroxide (NH₄OH) in a molar concentration of between 0.1 and 3.5 M and a solution of nucleic acids (NAs) in a concentration of between 0.01 and 0.50 µg/µl;
(ii) adding to the solution obtained in (i) tetra-ethyl-orthosilicate (TEOS) in a molar concentration of between 0.10 and 0.40M; and
(iii) stirring the solution obtained in (ii) between 100 and 1000 rpm for 1 to 3 hours at a temperature between 5 and 40°C, obtaining a colloidal suspension of silica particles comprising the nucleic acids encapsulated therein.

The terms "nucleic acid", "encapsulation" and "silica particle" have already been described in previous aspects of the present invention and apply equally to this inventive aspect, as well as all its preferred embodiments (alone or in combination).

The term "molarity" or "mol/L" or "M", used interchangeably in the present description, refers to the molar concentration (also called molarity), being a measure of the concentration of a solute in a solution, whether it is a molecular, ionic or atomic species, i.e., the ratio between the amount of solute (expressed in moles) per litre of solution or per unit of volume. It is routine practice for a person skilled in the art to determine the units of measurement of volume depending on the solute and the solvent, and calculate the molarity (M) of the solute and/or solvent of the solution based on the final volume.

As understood by a person skilled in the art, the nucleic acid solution comprises a solute, nucleic acids or NAs and a solvent. In another preferred embodiment of the method of the invention, the solvent of the nucleic acid solution of step (i) is deionised water (dH₂O) or Milli-Q water between 2 and 18M, or 16% v/v Tris EDTA (TE) buffer or ethanol, preferably in deionised water (dH₂O) or Milli-Q water.

In another preferred embodiment, the concentration of the nucleic acid solution of step (i) is between 0.01 and 0.50 µg/µl, preferably between 0.15 and 0.35 µg/µl. In another more preferred embodiment, the initial concentration of the nucleic acid solution (b) of step (i) is 0.16 µg/µl, 0.17 µg/µl, 0.18 µg/µl, 0.19 µg/µl, 0.20 µg/µl, 0.21 µg/µl, 0.22 µg/µl, 0.23 µg/µl, 0.24 µg/µl, 0.25 µg/µl, 0.26 µg/µl, 0.27 µg/µl, 0.28 µg/µl, 0.29 µg/µl, 0.3 µg/µl, 0.31 µg/µl, 0.32 µg/µl, 0.33 µg/µl or 0.34 µg/µl, preferably 0.225 µg/µl.

In the present invention, the nucleic acid solution of the method of the invention, is added in the pre-solidification liquid phase (step (i)), allowing nucleic acids to become encapsulated, once the silica particle is synthesised, inside the same. "Pre-solidification liquid phase" is understood herein as the mixture of components before the solidification of the silica particles. This solidification, in which the mixture of components gradually evolves towards a gel-like network, starts after adding the TEOS of step (ii).

As understood by a person skilled in the art, the volume of ethanol (EtOH) is calculated based on the concentration or volume of the rest of the components of the method of the invention and is added until the desired final volume is completed.

In another preferred embodiment, the molar concentration of ethanol is between 9M and 11M, that of NH₄OH is between 0.30M and 2M, that of dH₂O is between 9M and 11M and that of TEOS is between 0.15M and 0.35M.

In another more preferred embodiment, the molar concentration of ethanol is between 9.5M and 10.5M, that of NH₄OH is between 0.3M and 1.5M, that of dH₂O is between 9.5M and 10.5M and that of TEOS is between 0.2M and 0.3M.

In another even more preferred embodiment, in step (i) of the method of the invention, the molar concentration of EtOH is between 9.7 and 10 M, that of NH₄OH is 0.34M or 1.06M or 2.2M and that of dH₂O is 9.77M, and/or in step (ii) the molar concentration of TEOS is 0.25M.

In step (iii) of the method of the invention, stirring allows the solidification of the silica particles that encapsulate the nucleic acids inside the same to be completed, obtaining a colloidal suspension of silica particles that comprise the encapsulated nucleic acids. The terms "colloidal suspension", "colloidal system" or "colloidal dispersion", used interchangeably herein, refer to a system made up of two or more phases, normally a fluid (liquid or gas) phase and another disperse phase in the form of generally solid particles, specifically the particles of the invention.

It is routine practice for one skilled in the art to determine the stirring based on the total volume of the solution and other factors such as, for example, density, viscosity, tank or bioreactor dimensions, among others. Examples of type of stirring include, but are not limited to, magnetic stirring or mechanical stirring.

The term "magnetic stirring" used herein refers to stirring based on the rotation of a variable external magnetic field that induces a magnet introduced into the reaction solution to rotate, specifically for volumes less than 1 litre (1L). There are different types of magnetic stirring systems, such as vortex mixers or magnetic stirrers.

The term "mechanical stirring" used herein refers to stirring based on the rotation or movement of a fluid within a vessel, tank or reactor forced or carried out by mechanical means to acquire a circulatory movement inside a vessel, specifically with the use of motors. There are different types of mechanical stirring systems such as paddle stirrers, orbital stirrers, oscillating stirrers, rotary stirrers, vibrating stirrers, among others.

In a preferred embodiment, step (iii) is performed by magnetic stirring with a vortex stirrer or magnetic stirrer between 100 and 300 rpm, preferably between 150 and 200 rpm.

In another preferred embodiment, step (iii) is performed for 1.5 to 2.5 hours, more preferably for 2 hours.

In another more preferred embodiment, step (iii) of the method of the invention is performed at 15°C-30°C. In another more preferred embodiment, step (iii) is performed between 18 and 27°C, preferably between 20 and 25°C, more preferably at 22°C or 23°C.

In another preferred embodiment, the method of the invention may comprise an additional step, step (iv), where the particles obtained in step (iii) are washed with ethanol for 1 to 5 cycles, wherein each cycle comprises (i) centrifugation between 3500 rpm and 8000 rpm for 1 to 15 minutes and (ii) redispersion with ethanol and stirring for 20 to 80 seconds between 100 and 300 rpm.

In another more preferred embodiment, step (iv) of the method of the invention comprises 3 cycles wherein each cycle comprises (i) centrifugation between 5000 rpm and 65000 rpm for 3 to 10 minutes and (ii) redispersion with ethanol and stirring for 30 to 60 seconds between 150 and 200 rpm.

Specifically, the particles of the invention can be stored in ethanol, preferably in an 80 to 100% v/v ethanol solution, preferably 99% v/v,

By means of the method of the invention, the nucleic acids are integrated or encapsulated within the silica particle of the invention, allowing the surface of the particles to be functionalised for other purposes, such as, for example, for the targeting or protein coating of the particle of the invention.

The term "surface functionalisation" used herein refers to the process of adhering, combining, attaching or inserting functional groups, creating a stable attachment between them and the surface of the silica particle of the invention, to focus or direct the particles to a certain cell type, tissue or organ, to facilitate cellular incorporation of the particles or to synthesise a protein coating. Examples of functional groups include, but are not limited to, peptides, proteins, protein fragments, nucleotides, enzymes, antibodies, nanoantibodies, dendrimers, molecules targeting receptors (for example, folic acid, capsaicin, menthol) or aptamers, among others. The attachment between the functional group and the surface of the silica particle is established by electrostatic interactions, covalent bonds or Van der Waals interactions or hydrogen bonds.

Thus, in another preferred embodiment, the method of the invention may comprise an additional step, step (v), wherein the surface of the silica particle obtained by the method of the invention is functionalised.

In another more preferred embodiment, the surface of the particle is functionalised with at least one functional group that is selected from the list consisting of: peptides, proteins or protein fragments, nucleotides, enzymes, antibodies, dendrimers, molecules targeting receptors and aptamers.

It is routine practice for an expert in the field to use the techniques and methods of functionalising surfaces with functional groups. Examples of functionalisation techniques or methods include, but are not limited to: physical adsorption, electrostatic interactions, or hydrophobic interactions.

Likewise, the functional groups may comprise cell markers to allow visualisation of the particle of the invention. In another even more preferred embodiment, the functional groups also comprise at least one cell marker, preferably a fluorescent marker.

The term "cell marker" has already been described in previous aspects of the present invention and applies equally to this inventive aspect, as well as all its preferred embodiments (alone or in combination).

By means of the method of the invention described above, the inventors have developed silica particles comprising nucleic acids (NAs) encapsulated inside the silica particle.

Therefore, another aspect of the invention relates to a silica particle obtained by the method of the invention, where said particle comprises at least one nucleic acid encapsulated inside the same.

The particles of invention, as well as the particles obtained by the method of the invention, are stable and efficient in gene transfer, because the nucleic acids (NAs) are encapsulated inside the silica particle and are only released when the particle of the invention is dissolved.

Thus, another aspect of the present invention relates to the *in vitro* use of the particle (or the particle obtained by the method of the invention) or the composition of the invention in gene transfer.

In the present invention, the term "gene transfer" or "gene transduction" refers to the process whereby exogenous nucleic acids are transferred to host cells, wherein said nucleic acids are stabilised, incorporated and/or expressed in the host cell. In the present invention, the gene transfer system is the silica particle of the invention that the nucleic acid encapsulates inside the same and is released inside the host cell. The term *"in vitro* use" refers to use in a cell culture or in an isolated biological sample, i.e., it refers to use outside the human or animal body.

Nucleic acids encapsulated and released inside the host cell can be used in molecular biology applications, such as genetic engineering, for example, to manipulate the genes either by deleting, substituting, duplicating, inhibiting, silencing or inserting genetic material by means of gene editing technologies.

Specifically, by using NAs or proteins encoded by the encapsulated NAs, such as Dicer proteins, argonauts, RNase, miRNA or siRNA, gene expression is inhibited after gene transfer mediated by the particles of the invention, as described above.

Thus, a preferred embodiment of the invention refers to the *in vitro* use of the particle (or the particle obtained by the method of the invention) or the composition of the invention in gene silencing. The term "gene silencing" has already been described in previous aspects of the present invention and applies equally to this inventive aspect, as well as all its preferred embodiments (alone or in combination).

Furthermore, as described above, in addition to encapsulated NAs, the silica particle of the invention may comprise proteins forming gene editing systems such as, for example, CRISPR-Cas systems.

Thus, another aspect of the present invention relates to the *in vitro* use of the particle (or the particle obtained by the method of the invention) or of the composition of the invention in gene editing. The term "gene editing system" has already been described in previous aspects of the present invention and applies equally to this inventive aspect, as well as all its preferred embodiments (alone or in combination).

As described above, the particles of the invention can be used in cell marking.

Another aspect of the invention refers to the *in vitro* use of the particle (or the particle obtained by the method of the invention) or the composition of the invention in cell marking, preferably marking of the cell nucleus.

As used herein, the term "cell marking" refers to the exploitation of luminescent properties (for example, fluorescent properties) of certain molecules that are widely used in molecular biology to act as light signals that allow the visualisation of cells or regions thereof using imaging techniques, specifically with cell markers as described hereinbefore and which apply equally in this inventive aspect.

As described above, the particle of the invention can be used as a medicinal product. Therefore, another aspect of the invention relates to the particle or composition of the invention, or the particle obtained by the method of the invention, for use thereof as a medicinal product.

The term "medicinal product", as used herein, refers to any substance used for the prevention, diagnosis, alleviation, treatment or cure of diseases in a subject or that can be administered to the subject in order to restore, correct or modify the physiological functions thereof by exerting a pharmacological, immune or metabolic effect. In the context of the present invention, the medicinal product comprises the silica particle of the invention or alternatively a composition comprising at least the silica particle of the invention. For purposes of the present invention, the terms "medicinal product" and "pharmaceutical composition" are used synonymously.

Another aspect of the present invention relates to the use of the particle of the invention, of the particle obtained by means of the method of the invention or the composition of the invention, for manufacturing a medicinal product.

In a preferred embodiment, the medicinal product is a vaccine. Preferably, the vaccine is against SARS-CoV-2. The term "vaccine" has already been described in previous aspects of the present invention and applies equally to this inventive aspect, as well as all its preferred embodiments (alone or in combination).

As understood by a person skilled in the art, the silica particle of the invention (or the particle obtained by the method of the invention) can be used in protein/enzyme replacement therapy.

Therefore, another preferred embodiment relates to the particle of the invention (or the particle obtained by the method of the invention) or the composition of the invention for use as a medicinal product for protein/enzyme replacement therapy.

The term "protein/enzyme replacement therapy" refers in the present invention to a type of biological therapy that consists of delivering to the patient an exogenous protein that replaces the same protein in the patient's body, that presents a dysfunction or deficiency thereof. In the present invention, the exogenous protein is encoded by the encapsulated nucleic acid.

Another aspect of the present invention relates to the use of the particle of the invention, of the particle obtained by means of the method of the invention or the composition of the invention, for manufacturing a medicinal product for protein/enzyme replacement therapy.

Furthermore, as is known to a person skilled in the art, both the particle of the invention (or the particle obtained by the method of the invention) comprising a nucleic acid encoding a cell marker (or comprising a cell marker not encoded by a nucleic acid), as well as the composition of the invention, can be used as a contrast agent for observing cells. Thus, another aspect of the present invention relates to the use of the particle of the invention as a contrast agent for observing cells.

The term "contrast agent for observing cells" refers to a compound, element, composition, solution, mixture, biocompatible (non-toxic) substance that produces a detectable signal, capable of being monitored during injection in a subject by means of, for example, radiography or fluoroscopy.

### BRIEF DESCRIPTION OF THE FIGURES

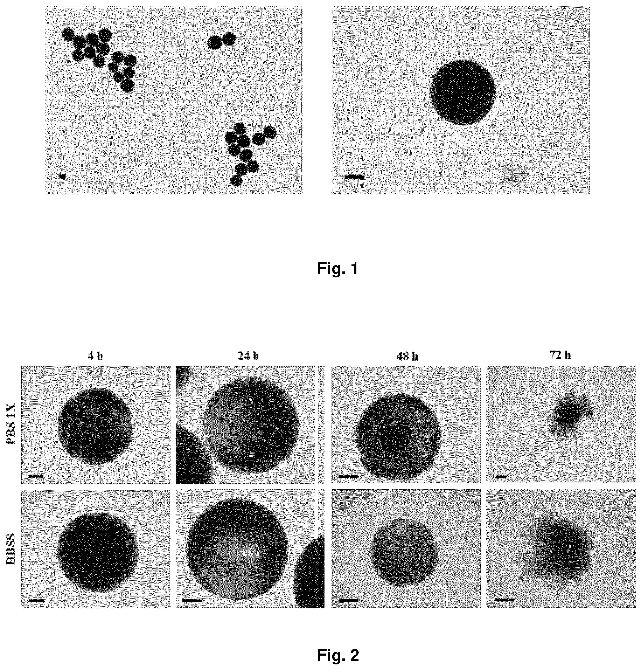

**Figure 1****.** TEM images of the DNA nanoparticles. Scale: 100 nm. Mean and standard deviation of the diameter: 388 ± 36 nm.
**Figure 2****.** TEM images of the solution of the silica particles *in vitro* in physiological saline conditions equivalent to the cell interior at times of 4h, 24h, 48h and 72h in PBS1X and in Hank's buffer saline solution (HBSS). Scale bar: 100 nm.

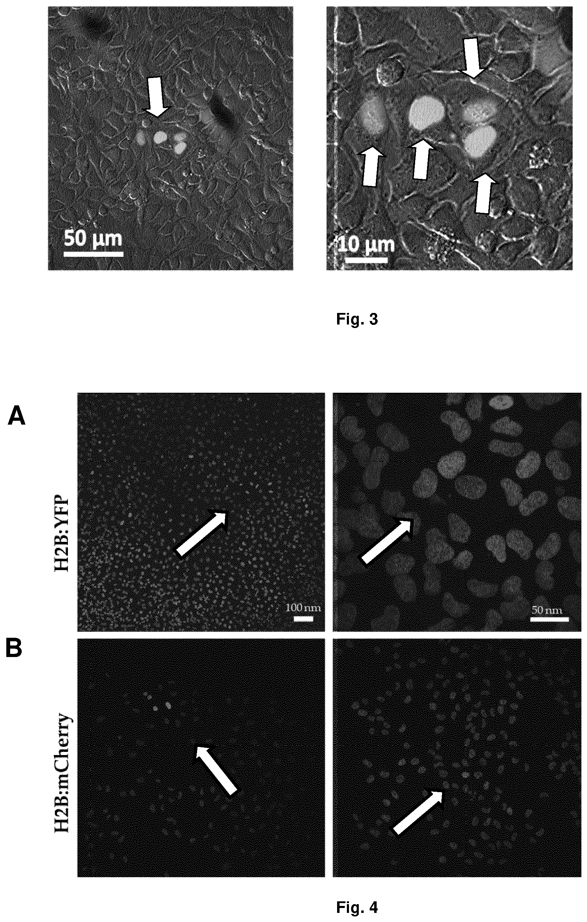

**Figure 3****.** Fluorescence microscopy images of human (HeLa) cells transfected with a silica particle containing NAs encoding GFP:NLS. Fluorescent nuclei are observed as a result of the expression of the gene of the nuclear fluorescent protein H2B:YFP (indicated by white arrows) encoded by the NAs carried by the particles. Marking with arrows of the nuclei that emit fluorescence.
**Figure 4****.** Confocal microscopy images of human cells (HeLa) incubated with the DNA nanoparticles after 72 h transfected with particles carrying 2 genes encoding 2 different fluorescent nuclear proteins. Stained nuclei of all the cells in the culture are observed (with Hoescht), and only some nuclei express the gene of the nuclear protein H2B:YFP (Figure 4A, indicated by white arrows). As a result of the correct transcription and translation of this protein, nuclei are observed showing the nuclear protein. The expression of another nuclear fluorescent protein, H2B:mCherry (Figure 4B; indicated by white arrows) encoded in the DNA carried by the second type of particles, is also observed.

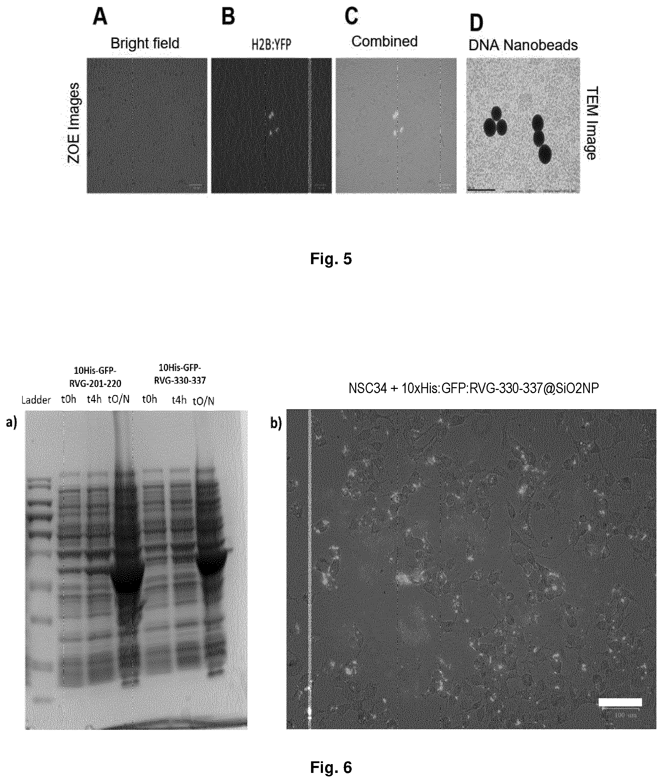

**Figure 5****.** Stability of the nanoparticles and their gene transfer capacity after 1 month of storage in ethanol at room temperature. A: Cell cultures are observed in bright field; B: The expression of the fluorescent protein in the nuclei of transfected cells; and C: images A and B combined. D: Transmission microscopy image showing the integrity of the particles after one month of storage.
**Figure 6. (a)** SDS-PAGE gel of two peptides belonging to the protein 10xHis:GFP:RVG-330-337. **(b)** Combined fluorescence microscopy image (10 His-GFP channel + bright field) of the motor neurons of the NSC34 line incubated with the silica nanoparticles functionalised with 10xHis:GFP:RVG-330-337 after 24 h. The fluorescence observed results from the coating of the particles with the fluorescent protein. Scale bar = 100 micrometres.

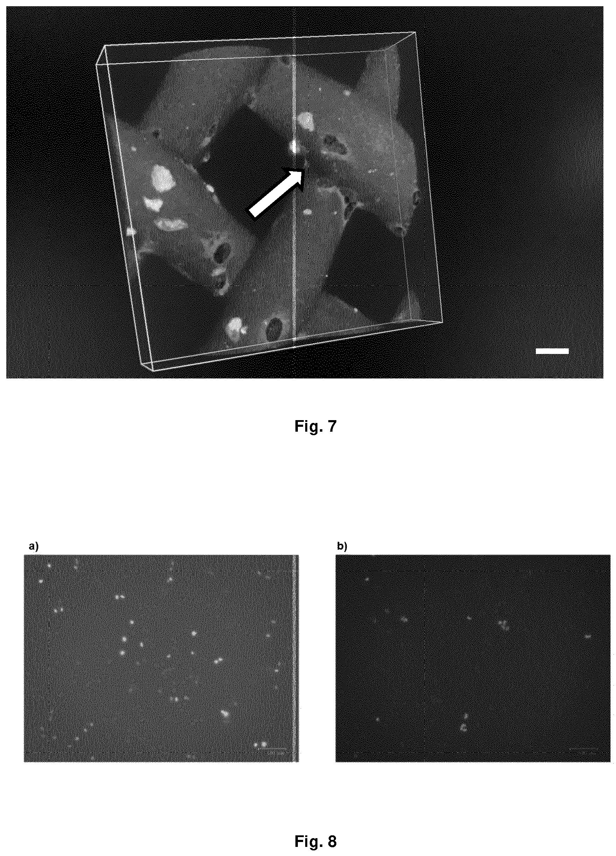

**Figure 7****.** 3D confocal image of a cellulose matrix where HeLa cells and motor neurons of the NSC34 line have been cultured. Nuclei were stained in Hoescht. These cells were incubated with DNA nanoparticles functionalised on their surface with the neuron targeting protein 10xHis:GFP:RVG-330-337. At 24 hours, NSC34 cells show gene expression of the fluorescent protein H2B:mCherry encoded by DNA encapsulated in silica particles. Scale bar = 100 micrometres.
**Figure 8****.** Fluorescence microscopy images of cells transfected with silica NPs with recombinant DNA encoding H2B:GFP. **(a)** Cells incubated with original NPs not treated with DNase. **(b)** Cells incubated with NPs pretreated with DNase. The fluorescence emission from the cell nuclei in both cases, associated with gene transfer and functional expression of H2B:GFP, indicates that the DNA is intact after nuclease treatment; therefore, it is deduced that it is encapsulated inside the NPs.

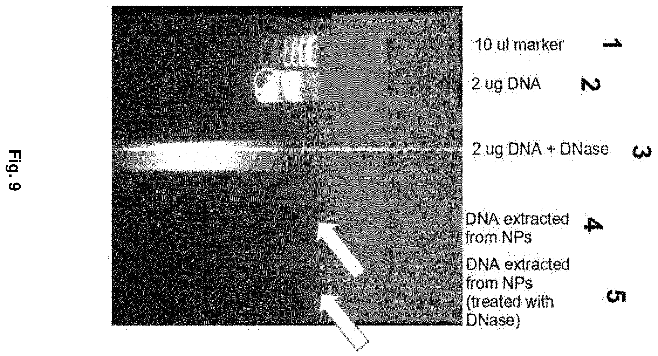

**Figure 9****.** Agarose gel showing size markers for the DNA in the gel (lane 1). In lane 2, the recombinant DNA encoding H2B:GFP used in encapsulation. In lane 3, that same unencapsulated DNA treated with DNase. In lane 4, the DNA extracted from the nanoparticles after incubation in PBS buffer. And in Lane 5, in DNA extracted from nanoparticles treated with DNase. The absence of any band in the DNA well treated with DNase (lane 3) indicates its degradation and, therefore, correct functioning of the DNase used in the assays. The arrows show how the amount of DNA extracted from untreated NPs and NPs treated with Dnase is very similar, suggesting that they protect the DNA encapsulated inside the same.

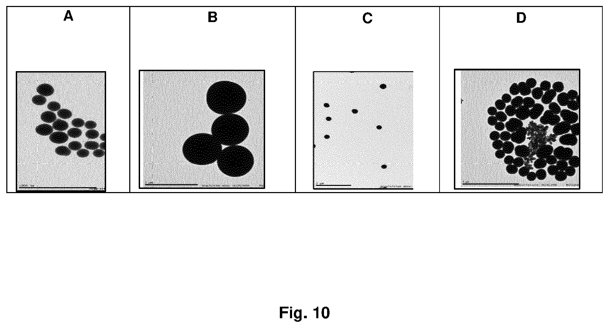

**Figure 10****.** TEM images of the synthesis of DNA silica particles inside the produced using different molarities of the precursor elements and plasmids: A) H2B:YFP, B) H2B:YFP, C) H2B:mCherry and D) H2B-YFP.

### EXAMPLES

Next, the invention will be illustrated by means of assays carried out by the inventors which demonstrate the effectiveness of the invention.

### 1. MATERIALS AND METHODS

### 1.1. Obtaining silica particles with encapsulated nucleic acids.

To make silica (Si₂) particles or spheres, the inventors used ethanol (EtOH, 99%), Milli-Q water, solution of nucleic acids (NAs), specifically of recombinant DNA, in a DNA concentration in synthesis of between 0.075 and 3 µgr/µl), ammonium hydroxide (NH₄OH) and tetra-ethyl-orthosilicate (98% TEOS, a precursor of conventional silica). The extracted NAs were 100% dissolved in Milli-Q water, or alternatively they can be dissolved in Tris-EDTA buffer (TE buffer), finally at 16% in Milli-Q water or in ethanol, so that it does not affect the synthesis of silica particles. Some of the NA proportions used are shown in Table 1.

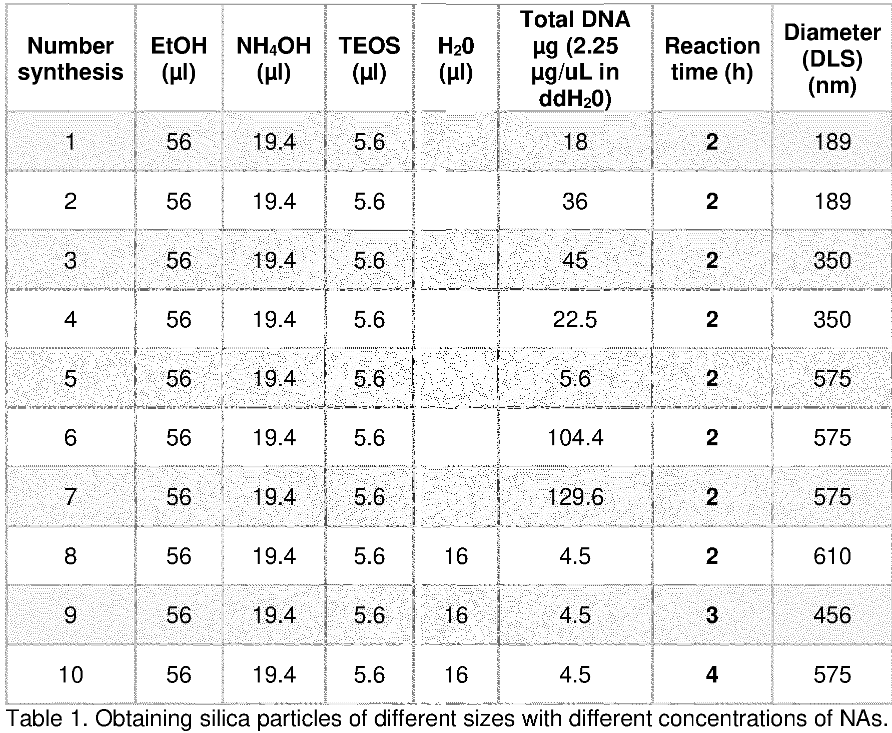

The synthesis of silica spheres is performed following the Stöber method (Stöber, W., et al., J. Colloid Interface Sci. 26 (1), 62-69 (1968)). For this, a solution of EtOH:H₂O-DNA:NH₄OH (59:18:19.4 µl) is used, corresponding to a molar concentration of EtOH is 9.91M, that of NH₄OH is 2.02M and that of dH₂O is 9.77M, and/or in step (ii), the molar concentration of TEOS is 0.25M, in a closed eppendorf to which 5.6 µl of TEOS was added, which was vigorously stirred with a vortex at 150-200 rpm at ambient temperature at about 22°C for 2 hours, obtaining a white cloudy suspension. The TEOS must be added quickly to the reaction to avoid evaporation of NH₄OH (closed reaction).

The monodisperse spheres were washed with ethanol by means of 3 cycles of redispersion/centrifugation (between 5000 and 6500 rpm for 3 to 10 minutes, preferably at 6500 rpm for 3 min or at 5000 rpm for 10 min), and finally they were redispersed in 100 µl of EtOH. The particle solution concentration was about 0.0174 g/ml. The silica nanoparticles (NPs) were examined by electron microscopy, obtaining silica spheres of about 300 to 500 nm in diameter.

The inventors used DNA encoding for a reporter protein, specifically for fluorescent proteins, as NAs for encapsulation in silica NPs.

The inventors synthesised the green fluorescent protein (GFP), directed to the nucleus by means of binding to a nuclear localisation sequence (NLS), giving rise to GFP:NLS recombinant DNA.

Furthermore, the inventors also used recombinant DNAs that expressed the recombinant protein H2B:YFP and the H2B:mCherry protein with different tested molarities of the precursor elements of the silica particles of the invention, wherein the reaction time and temperature are not modified in this type of reaction, only the NH4OH:H2O:TEOS ratio is modified to change the different sizes (Table 2). These changes are not proportional, i.e., the fact that the concentration of one of them is increased does not mean that the size of the particles will increase.

**Table 2. Different synthesised samples. Notes: DNA was dissolved in H₂O; and the EtOH is COMPLETED to the desired final volume.**

| | **Sample with H2B:YFP** | **Sample with H2B:YFP** | **Sample with H2B:mCherry** | **Sample with H2B:YFP** |
|---|---|---|---|---|
| **H₂O [M]** | 17.4 | 3.32 | 5.4 | 4.71 |
| **NH₄OH (aqueous NH₃) [M]** | 0.34 | 1.06 | 0.35 | 1.06 |
| **TEOS [M]** | 0.25 | 0.25 | 0.25 | 0.25 |
| **Plasmid** | H2B:YFP | H2B:YFP | H2B:mCherry | H2B-YFP |
| **Final [DNA] [pmol]** | 2.1 | 1.7 | 7 | 13.7 |
| **Total synthesis volume (µl)** | 100 | 100 | 100 | 1000 |
| **Size (nm)** | 200 | 500-700 (polydisperse) | 284 | 300-500 (polydisperse) |

### 1.2. Obtaining functionalised silica particles on the surface

The inventors used a recombinant protein, 10xHis:GFP:RVG-330-337, as a ligand to coat the surface of these particles and direct the particles to neurons, by means of the neuronal receptor binding peptide RVG-330-337.

The encapsulated recombinant DNA containing the insert encoding the green fluorescent proteins GFP with a nuclear localisation sequence (GFP:NLS), was amplified in recombinant bacteria. After bacterial lysis, DNA was purified using a commercial DNA purification column. This was eluted from the column in distilled water. Its concentration was calculated following standard absorbance methods at a wavelength of 260 nm (A260). The DNA was finally resuspended at a concentration of 2.5 µg/µl in water.

Said recombinant protein was purified by means of affinity purification techniques using nmicale columns following the standard protocol of Padín-González, *et al.,* being overexpressed for 112-16 hours (O/N).

### 1.3. Characterisation of silica nanoparticles

The samples obtained from the synthesis of nanoparticles were deposited (diluted in ethanol) on carbon-coated copper grids to be observed under the transmission electron microscope (TEM). Fluorescence microscopy was also used to observe the cells through an epifluorescence microscope.

To evaluate the capacity of silica particles as vectors of biological agents, such as nucleic acids, a trial was carried out *in vitro* dissolving them in 1X PBS and HBSS. The particles obtained diluted in 1X PBS and HBSS were incubated at 37°C for 72 h at different times (4, 24, 48 and 72 hours) by means of centrifugation of the nanoparticles and subsequent visualisation in the TEM.

### 1.4. Gene transfer of particles in cell cultures

To evaluate the gene transfer capacity of NPs, the silica particles were incubated with the encapsulated recombinant DNA (GFP:NLS, H2B:YFP and H2B:mCherry) with HeLa cells (human cervical cancer cells) and/or NSC34 neuronal cells, both grown in MEM medium (Biowhittaker^{™}) with 10% FBS (fetal bovine serum) under standard conditions.

These cell lines were plated (50,000 cells/well in a 24-well plate) at a density of 60% and incubated for 8 h to allow adherence. Then, the silica particles containing the recombinant DNA were added at a concentration of 0.348 µgr/µl in the culture medium.

Subsequently, the culture plates were centrifuged (4000 g, 6 min) to force the entry of the silica particles with the encapsulated DNA into the cell cytoplasm. In the case of the NSC34 neuronal line when the added particles were coated with ligand 10xHis:GFP:RVG-330-337, it was not necessary to centrifuge because the particles enter through a receptor-mediated mechanism targeting the neurons.

They were incubated for 16 h, subsequently washed with HBSS, and fresh medium was added. This was done again at 24 and 36 h. These washes were carried out to reduce the amount of silicon in the culture medium and favour the dissolution of the particles in less time.

Once the desired level of expression has been reached, cells were fixed with 4% paraformaldehyde and stained with Hoescht 33258, both from Sigma Aldrich. The inventors verified by means of fluorescence microscopy the presence of fluorescent nuclei that demonstrated the correct gene expression at the protein level from the DNA encapsulated in the NPs. Confocal microscopy images were taken with a Nikon A1R microscope.

Furthermore, the inventors encapsulated recombinant H2B:mCherry DNA in nanoparticles, which in turn were coated with the targeting protein 10xHis:GFP:RVG-330-337 and added to a coculture of HeLa and NSC34 neurons, previously incubated in a cellulose matrix in a 6-well plate.

### 1.5. DNase assays

To check the encapsulation of nucleic acids inside the silica particles, the inventors carried out tests with DNase. To that end, silica particles containing recombinant DNA encoding H2B:GFP were subjected to 5.4 µl of DNase, 5 µl PBS and 1 µl DNA (2 µg) for 1h 20 min. Subsequently, the inventors incubated HeLa-type cells with the particles after treatment with DNase, with untreated particles (negative control) and with control particles (not treated with DNase) following the protocol described in section 1.3 above. Gene expression results were visualised by means of fluorescence microscopy.

Furthermore, as a control of the functioning of the DNase used, the inventors treated the same unencapsulated recombinant DNA with DNase following the same method. To visualize the result of the NAs, electrophoresis was performed in 1% agarose gel with ethidium bromide at a voltage of 100 volts for 20 minutes. The following samples were added to different wells: 10 µl of a marker, unencapsulated recombinant DNA (2 µg) and unencapsulated recombinant DNA (2 µg) incubated with DNase; and the DNA extracted by means of dissolving the untreated particles and the particles treated with DNase. In both cases an almost identical band of DNA is detected. This indicates that the treated particles are DNase and protect the NAs encapsulated inside the same.

### 2. RESULTS

### 2.1. Characterisation of nanoparticles and dissolution of silica

By means of transmission electron microscopy (TEM), it was determined that their structure is spherical and monodisperse, as can be observed in Figure 1.

The particles diluted in PBS1X and HBSS were incubated for different times (4, 24, 48 and 72 hours). By means of visualisation in the TEM (Figure 2), it is observed how the silica particles begin to dissolve in cellular media over time. The comparison of the morphology of the particles at different times showed a progressive decrease and loss of spherical shape (Figure 2) which demonstrated that silica particles are not only capable of penetrating cells, but also dissolving under physiological chemical conditions similar to those of the cell cytoplasm, locally releasing the nucleic acids introduced in the mixture.

Furthermore, by means of transmission electron microscopy (TEM), the structure of the silica particles was determined with different tested molarities of the precursor elements and plasmids, as shown in Figure 10, A)H2B:YFP, B) H2B:YFP, C) H2B:mCherry and D) H2B-YFP.

### 2.2 Gene transfer in HeLa cells with GFP:NLS, H2B: YFP and H2B:mCherry

To evaluate the gene transfer capacity of the nanoparticles, HeLa cells were plated with the NPs containing the recombinant DNA with the GFP:NLS insert. At 36/48h, the presence of fluorescent nuclei was verified by means of fluorescence microscopy that demonstrated the correct gene expression at the protein level from the DNA encapsulated in the NPs (Figure 3).

The presence of fluorescent nuclei of HeLa cells incubated with NPs was verified by means of fluorescence microscopy with H2B:mCherry and with H2B:YFP, demonstrating the correct gene expression at the protein level from the DNA encapsulated in the NPs after 72 h. Figure 4 shows Hoescht-stained nuclei, nuclei expressing the gene of the H2B:YFP protein (Figure A, arrows), nuclei expressing the gene of the H2B:mCherry protein (Figure B, arrows).

### 2.3. Stability of nanoparticles over time

The silica nanoparticles with the encapsulated DNA were stored for 1 month at room temperature in ethanol. Next, they were added to HeLa cells as described above to check for the effectiveness of gene expression (Figure 5). Gene expression one month after the nanoparticles were synthesised can be seen by means of fluorescence microscopy images. The nanoparticles remain stable in ethanol at ambient temperature, as can be seen in the TEM image of Figure 5.

### 2.4. Gene transfer in NSC34 neuronal cells with particles functionalised with the recombinant protein ligand 10xHis:GFP:RVG-330-337

The recombinant protein was purified by means of affinity purification techniques using nmicale columns (Figure 6a), being overexpressed for 112-16 hours (O/N). As shown in Figure 6b, by combined fluorescence microscopy (10 His-GFP channel + bright field) of NSC34 incubated with nanoparticles functionalised with the neuronal ligand (10xHis:GFP:RVG-330-337@SiO2NP) after 24 h, expression of the peptide RVG-330-337 in NSC34 is observed.

In Figure 7, the expression of the H2B:mCherry protein (arrow) can be observed in the nucleus of neurons, showing the ability to encapsulate in these silica nanoparticles and to specifically target neurons with this ligand to perform targeted therapy, for example, maintaining gene expression.

### 2.5. Encapsulation of DNA inside the silica particles

To evaluate the encapsulation of nucleic acids inside the silica particles, the inventors carried out tests with DNase.

Figure 8 shows, by means of fluorescence microscopy, the expression of the H2B:GFP protein, both in cells transfected with silica particles without DNase treatment (Figure 8a) and in cells transfected with particles treated with DNase (Figure 8b).

Figure 9 demonstrates the effectiveness of the DNase used in the test. Recombinant H2B:GFP DNA treated with DNase degrades correctly, as seen in the third well of the gel (2 µg DNA + DNase), unlike the same recombinant DNA without DNase treatment, as observed in the second well (2 µg DNA) where the band corresponding to the molecular weight of recombinant H2B:GFP DNA appears. In the following lanes it is observed that the DNA extracted from untreated particles and particles treated with DNase is similar, confirming that the DNA is protected by the particle.

With this test, the inventors demonstrated that the nucleic acids are encapsulated inside the silica particles of the invention, being protected (for example, from enzymes that degrade NAs, such as DNase), and that they are only released when these particles are dissolved inside the cell. This demonstrates its effectiveness in gene transfer, allowing the use thereof in cell marking or therapeutic use, for example, in protein/enzyme replacement therapy or in the administration of vaccines.

## Claims

1. A method for producing a silica particle **characterised in that** it comprises at least one nucleic acid encapsulated inside said particle, comprising the following steps:
(i) dissolving ethanol (EtOH) in a molar concentration of between 8 and 12 mol/L (M), ammonium hydroxide (NH₄OH) in a molar concentration of between 0.1 and 3.5 M and a solution of nucleic acids (NAs) in a concentration of between 0.01 and 0.50 µg/µl;
(ii) adding to the solution obtained in (i) tetra-ethyl-orthosilicate (TEOS) in a molar concentration of between 0.10 and 0.40M; and
(iii) stirring the solution obtained in (ii) between 100 and 1000 rpm for 1 to 3 hours at a temperature between 5 and 40°C, obtaining a colloidal suspension of silica particles comprising the nucleic acids encapsulated therein.

2. The method according to claim 1, wherein the solvent of the nucleic acid solution of step (i) is deionised water (dH₂O) between 2 and 18M, Milli-Q water, 16% v/v Tris EDTA (TE) buffer or ethanol, preferably dH₂O or Milli-Q water.

3. The method according to claim 1 or 2, wherein the concentration of the nucleic acid solution of step (i) is between 0.15 and 0.35 µg/µl, preferably 0.225 µg/µl.

4. The method according to any one of claims 1 to 3, wherein in step (i) the molar concentration of EtOH is between 9.7M and 10M, that of NH₄OH is 0.34 or 1.06 or 2.02M and that of dH₂O is 9.77M and/or in step (ii) the molar concentration of TEOS is 0.25M.

5. The method according to any one of claims 1 to 4 further comprising a step (iv), wherein the particles obtained in step (iii) are washed with ethanol for 1 to 5 cycles, wherein each cycle comprises (i) centrifugation between 3500 rpm and 8000 rpm for 1 to 15 minutes and (ii) redispersion with ethanol and stirring for 20 to 80 seconds between 100 and 300 rpm.

6. The method according to any one of claims 1 to 5, wherein step (iii) is performed for 2 hours.

7. The method according to any one of claims 1 to 6, wherein step (iii) is performed at between 15°C and 30°C, preferably at 22°C.

8. The method according to any one of claims 1 to 7 further comprising a step (v), wherein the surface of the silica particle is functionalised with at least one functional group that is selected from the list consisting of: peptides, proteins or protein fragments, nucleotides, enzymes, antibodies, dendrimeric nanoantibodies, molecules targeting receptors and aptamers.

9. A silica particle **characterised in that** it comprises at least one nucleic acid encapsulated inside said particle, obtained by the method according to any one of claims1 to 8.

10. *In vitro* use of the particle according to of claim 9 in gene transfer or gene editing.

11. *In vitro* use of the particle according to any of claims 9 for cell marking.

12. The particle according to claim 9 for use as a medicinal product.

13. The particle for use according to claim 12, wherein the medicinal product is a vaccine.

14. The particle according to claim 9 for use as a medicinal product for protein/enzyme replacement therapy.

15. Use of the particle according to claim 9 as a contrast agent for the observation of cells.

## Patentansprüche

1. Verfahren zur Herstellung eines Kieselsäureteilchens, **dadurch gekennzeichnet, dass** es mindestens eine innerhalb des Teilchens verkapselte Nukleinsäure umfasst, umfassend die folgenden Schritte:
(i) Auflösen von Ethanol (EtOH) in einer molaren Konzentration zwischen 8 und 12 mol/l (M), Ammoniumhydroxid (NH₄OH) in einer molaren Konzentration zwischen 0,1 und 3,5 M und einer Lösung von Nukleinsäuren (NAs) in einer Konzentration zwischen 0,01 und 0,50 µg/µl;
(ii) Hinzufügen von Tetraethylorthosilikat (TEOS) in einer molaren Konzentration zwischen 0,10 und 0,40 M zu der in (i) erhaltenen Lösung; und
(iii) Rühren der in (ii) erhaltenen Lösung bei 100 bis 1000 U/min für 1 bis 3 Stunden bei einer Temperatur zwischen 5 und 40 °C, wobei eine kolloidale Suspension von Kieselsäureteilchen erhalten wird, die die darin verkapselten Nukleinsäuren umfasst.

2. Verfahren nach Anspruch 1, wobei das Lösungsmittel der Nukleinsäurelösung aus Schritt (i) entionisiertes Wasser (dH₂O) zwischen 2 und 18 M, Milli-Q-Wasser, 16 % v/v Tris-EDTA (TE)-Puffer oder Ethanol ist, vorzugsweise dH₂O oder Milli-Q-Wasser.

3. Verfahren nach Anspruch 1 oder 2, wobei die Konzentration der Nukleinsäurelösung aus Schritt (i) zwischen 0,15 und 0,35 µg/µl, vorzugsweise 0,225 µg/µl beträgt.

4. Verfahren nach einem der Ansprüche 1 bis 3, wobei in Schritt (i) die molare Konzentration von EtOH zwischen 9,7 M und 10 M liegt, die von NH₄OH 0,34 oder 1,06 oder 2,02 M beträgt und die von dH₂O 9,77 M beträgt und/oder in Schritt (ii) die molare Konzentration von TEOS 0,25 M beträgt.

5. Verfahren nach einem der Ansprüche 1 bis 4, ferner umfassend einen Schritt (iv), wobei die in Schritt (iii) erhaltenen Teilchen 1 bis 5 Zyklen lang mit Ethanol gewaschen werden, wobei jeder Zyklus (i) Zentrifugation zwischen 3500 U/min und 8000 U/min für 1 bis 15 Minuten und (ii) Redispersion mit Ethanol und Rühren für 20 bis 80 Sekunden bei 100 bis 300 U/min umfasst.

6. Verfahren nach einem der Ansprüche 1 bis 5, wobei Schritt (iii) 2 Stunden lang durchgeführt wird.

7. Verfahren nach einem der Ansprüche 1 bis 6, wobei Schritt (iii) bei zwischen 15 °C und 30 °C, vorzugsweise bei 22 °C, durchgeführt wird.

8. Verfahren nach einem der Ansprüche 1 bis 7, ferner umfassend einen Schritt (v), wobei die Oberfläche der Kieselsäureteilchen mit mindestens einer funktionellen Gruppe funktionalisiert wird, die aus der Liste ausgewählt ist, bestehend aus: Peptiden, Proteinen oder Proteinfragmenten, Nukleotiden, Enzymen, Antikörpern, dendrimerischen Nanoantikörpern, Molekülen, die auf Rezeptoren abzielen, und Aptameren.

9. Kieselsäureteilchen, **dadurch gekennzeichnet, dass** es mindestens eine in dem Teilchen verkapselte Nukleinsäure umfasst, die durch das Verfahren nach einem der Ansprüche 1 bis 8 erhalten wird.

10. In-vitro-Verwendung des Teilchens nach Anspruch 9 beim Gentransfer oder bei der Genom-Editierung.

11. In-vitro-Verwendung des Teilchens nach einem der Ansprüche 9 zur Zellmarkierung.

12. Teilchen nach Anspruch 9 zur Verwendung als Arzneimittel.

13. Teilchen zur Verwendung nach Anspruch 12, wobei das Arzneimittel ein Impfstoff ist.

14. Teilchen nach Anspruch 9 zur Verwendung als Arzneimittel für die Protein- /Enzymersatztherapie.

15. Verwendung des Teilchens nach Anspruch 9 als Kontrastmittel zur Beobachtung von Zellen.

## Revendications

1. Procédé de production d'une particule de silice **caractérisé en ce qu'**il comprend au moins un acide nucléique encapsulé à l'intérieur de ladite particule, comprenant les étapes suivantes :
(i) dissoudre de l'éthanol (EtOH) à une concentration molaire entre 8 et 12 mol/L (M), de l'hydroxyde d'ammonium (NH₄OH) à une concentration molaire entre 0,1 et 3,5 M et une solution d'acides nucléiques (AN) à une concentration entre 0,01 et 0,50 µg/µl ;
(ii) ajouter à la solution obtenue à (i) de l'orthosilicate de tétraéthyle (TEOS) à une concentration molaire entre 0,10 et 0,40 M ; et
(iii) agiter la solution obtenue à (ii) entre 100 et 1 000 tr/min pendant 1 à 3 heures, à une température entre 5 et 40 °C, obtenant une suspension colloïdale de particules de silice comprenant les acides nucléiques encapsulés à l'intérieur.

2. Procédé selon la revendication 1, dans lequel le solvant de la solution d'acide nucléique de l'étape (i) est de l'eau déionisée (dH₂O) entre 2 et 18 M, Eau Milli-Q, un tampon Tris-EDTA (TE) à 16 % v/v ou de l'éthanol, de préférence du dH₂O ou de l'eau Milli-Q.

3. Procédé selon la revendication 1 ou 2, dans lequel la concentration de la solution d'acide nucléique de l'étape (i) est entre 0,15 et 0,35 µg/µl, de préférence 0,225 µg/µl.

4. Procédé selon l'une quelconque des revendications 1 à 3, dans lequel à l'étape (i) la concentration molaire d'EtOH est entre 9,7 M et 10 M, celle de NH₄OH est de 0,34, 1,06 ou 2,02 M et celle de dH₂O est de 9,77 M et/ou, à l'étape (ii), la concentration molaire de TEOS est de 0,25 M.

5. Procédé selon l'une quelconque des revendications 1 à 4, comprenant en outre une étape (iv), dans lequel les particules obtenues à l'étape (iii) sont lavées à l'éthanol pendant 1 à 5 cycles, chaque cycle comprenant (i) une centrifugation entre 3 500 et 8 000 tr/min pendant 1 à 15 minutes et (ii) une redispersion avec de l'éthanol et une agitation pendant 20 à 80 secondes entre 100 et 300 tr/min.

6. Procédé selon l'une quelconque des revendications 1 à 5, dans lequel l'étape (iii) est mise en œuvre pendant 2 heures.

7. Procédé selon l'une quelconque des revendications 1 à 6, dans lequel l'étape (iii) est mise en œuvre à des températures entre 15 °C et 30 °C, de préférence à 22 °C.

8. Procédé selon l'une quelconque des revendications 1 à 7, comprenant en outre une étape (v), dans lequel la surface de la particule de silice est fonctionnalisée avec au moins un groupe fonctionnel qui est choisi parmi la liste constituée par : des peptides, des protéines ou des fragments de protéines, des nucléotides, des enzymes, des anticorps, des nano-anticorps dendrimères, des molécules ciblant des récepteurs et des aptamères.

9. Particule de silice **caractérisée en ce qu'**elle comprend au moins un acide nucléique encapsulé à l'intérieur de ladite particule, obtenue par le procédé selon l'une quelconque des revendications 1 à 8.

10. Utilisation *in vitro* de la particule selon la revendication 9 dans le transfert de gènes ou l'édition de gènes.

11. Utilisation *in vitro* de la particule selon l'une quelconque des revendications 9 pour le marquage cellulaire.

12. Particule selon la revendication 9 destinée à être utilisée comme médicament.

13. Particule destinée à être utilisée selon la revendication 12, dans laquelle le médicament est un vaccin.

14. Particule selon la revendication 9 destinée à être utilisée comme médicament pour une thérapie de remplacement protéique ou enzymatique.

15. Utilisation de la particule selon la revendication 9 en tant qu'agent de contraste pour l'observation de cellules.
